# EUROPEAN PATENT SPECIFICATION

(11) **EP 0 531 708 B1**
(45) Date of publication and mention of the grant of the patent: **02.04.1997**
(21) Application number: 92113274.2
(22) Date of filing: 04.08.1992
(51) Int. Cl.: C12P 25/00, C12N 1/20

(54) **Process for producing riboflavin by fermentation**
Verfahren zur Herstellung von Riboflavin durch Fermentation
Procédé de production de riboflavine par fermentation

(30) Priority: 09.09.1991 JP 227864/91
(43) Date of publication of application: 17.03.1993
(73) Proprietor: Ajinomoto Co., Inc., Tokyo 104 (JP)
(72) Inventor: Usui, Naoki, c/o Central Research Lab., Kawasaki-shi, Kanagawa-ken (JP); Yamamoto, Yoko, c/o Central Research Lab., Kawasaki-shi, Kanagawa-ken (JP); Nakamatu, Tuyoshi, c/o Central Research Lab., Kawasaki-shi, Kanagawa-ken (JP)
(74) Representative: Strehl Schübel-Hopf Groening & Partner

(56) References cited:
- BE-A- 890 917
- FR-A- 2 204 687
- FR-A- 2 546 907
- US-A- 4 165 250

## Description

The present invention relates to a process for producing riboflavin by fermentation. Riboflavin is a substance important as a medicine, an additive to feed, a colorant for food, or the like.

Among hitherto known processes for producing riboflavin by fermentation are those in which Eremothecium ashbyii, Ashbya gossypii, Candida flalerii or Bacillus subtilis is cultured in a carbohydrate-containing medium and riboflavin is accumulated in the medium (Progress Industrial Microbiology, Vol. 1, p. 139 (1959); Belgian Patent No. 890,917; Japanese Patent Publication No. 10,155/78 and French application FR-A-2 204 687). Microorganisms used in those processes, however, accumulate riboflavin only at a low concentration or produce the compound only slowly. They are therefore not satisfactory as a method for commercially producing riboflavin.

It is an object of the present invention to obtain a microorganism having an improved riboflavin-producing capability and to provide a process for producing riboflavin through an efficient, inexpensive fermentation.

In order to develop a process for effectively producing riboflavin by fermentation, the present inventors have conducted intensive investigations and, as a result, have found that the above objects can be met by mutants which belong to the species Bacillus subtilis, having a decreased activity of liberating phosphoric acid from 5'-guanylic acid (hereinafter referred to as "5'-GMP") of not higher than 0.081 µmol/min/mg-protein and have the ability of producing riboflavin. The present invention has been completed on the basis of the above finding.

Accordingly, there is provided by the present invention a process for producing riboflavin by fermentation which comprise culturing in a liquid fermentation medium a mutant which belongs to the species Bacillus subtilis, having a decreased activity of liberating phosphoric acid from 5'-GMP of not higher than 0.081 µmol/min/mg-protein and having the ability of producing riboflavin; accumulating riboflavin in the medium; and recovering the riboflavin from the medium.

In the present invention, there is used a microorganism which belongs to the species Bacillus subtilis, has lowered activity of liberating phosphoric acid from 5'-GMP of not higher than 0.081 µmol/min/mg-protein and is capable of producing riboflavin. Mutants having a further improved riboflavin-producing capability can be obtained by furnishing such a microorganism with other properties (e.g., resistance to nucleic acid base analogues) known to be effective for the enhancement of riboflavin-producing ability (see Japanese Patent Publication No. 10, 155/78).

As an example of the mutant usable in the present invention, mention may be made of the following:
Bacillus subtilis AJ12644 (FERM BP-3855)
(This strain requires adenine, is GMP reductase deficient, is resistant to (purine + azaxanthine) and has lowered activity of liberating phosphoric acid from 5'-GMP)

Strains belonging to the species Bacillus subtilis can be used as a parent for obtaining such a mutant. As a specific example, mention may be made of the following riboflavin-producing bacterium Bacillus subtilis AJ12643 (FERM BP-3856) (This strain requires adenine, is GMP reductase deficient and is resistant to (purine + azaxanthine).).

Mutants according to the invention can be obtained by applying to such a parent strain a conventional mutation-inducing technique, for example, irridation by X-rays or ultraviolet rays and contact with a mutagenic agent, such as N-methyl-N'-nitrosoguanidine or the like.

An example of experiment for obtaining such a mutant is shown hereinbelow.

Into a liquid nutrient medium (pH 7.0) containing 2.5% of soluble starch, 0.3% of yeast extract, 0.3% of polypeptone and 0.1% of sodium chloride was inoculated a riboflavin-producing Bacillus subtilis AJ12643 (FERM BP-3856 ), which is an adenine-requiring microorganism derived from Bacillus subtilis ATCC 13952, deficient in GMP reductase and resistant to (purine + 8-azaxanthine). The microorganism was shake cultured at 34 °C for 16 hours, and 1 ml of the resulting culture was inoculated into 4 ml of nutrient medium having the same composition as above and shake cultured at 34 °C. At the time when the turbidity of the culture at 562 nm increased to 0.8, the cell bodies were collected by centrifugation and washed with 50 mM of phosphate buffer (pH 7.0). The washed cell bodies were suspended in the same buffer (5 ml), and 5 ml of 50 mM phosphate buffer (pH 7.0) containing 500 µg of N-methyl-N'-nitro-N-nitrosoguanidine was then added thereto. The resulting suspension was allowed to stand under ice-cooling for 40 minutes. The thus treated cell bodies were washed with the same buffer and then redispersed in 5 ml of the same buffer and then smeared on an agar plate medium containing the same nutrient as above. After being cultured at 34 °C for 2 days, the growth of colonies was confirmed. Thereafter, a filter paper soaked with disodium 4-nitrophenyl phosphate solution/50 mM tris buffer (pH 8.8) was placed on the plate, and the plate and the filter paper were allowed to stand at room temperature for 5 minutes. Since colonies of strains having a strong activity of liberating phosphoric acid from 5'-GMP are colored yellow, strains having a lowered activity of liberating phosphoric acid from 5'-GMP were selected as colonies developing only a weak yellow color.

The parent strain (Bacillus subtilis AJ12643) and the variants obtained as above were separately inoculated on liquid nutrient media having the above-described composition and shake cultured at 34 °C for 16 hours. The grown cell bodies were collected by centrifugation and washed with physiological salt solution. The cell bodies were then suspended and treated by a ultrasonic homogenizer at an output of 200 W for 7 minutes. The resulting solution containing the homogenized cell bodies was added to a solution of 40 mM (final concentration) of 5'-GMP in 200 mM MOPS buffer (pH 7.0), and the reaction was allowed to proceed at 34 °C for 2 hours. The activity of the strains for liberating phosphoric acid from 5'-GMP was determined by quantitatively analyzing guanosine by means of high performance liquid chromatography [column, CPK-08 produced by Mitsubishi Kasei Corp.; eluent, 3% lithium formate (pH 4.75);detection, UV at the wavelength of 260 nm]. In a separate test, the strains were inoculated into the above-described liquid nutrient medium (4 ml) and cultivated at 34 °C for 16 hours. The resulting cultures were inoculated into a riboflavin-production medium having the composition set forth in Table 1 (quantity of inoculation = 5%) and cultivated at 34 °C for 3 days, and the riboflavin-producing capability of the strains was determined.

**Table 1**

| Components | Concentration |
|---|---|
| Glucose | 80 g/l |
| NH₄Cl | 15 g/l |
| KH₂PO₄ | 0.2 g/l |
| MgSO₄·7H₂O | 0.4 g/l |
| Fe²⁺ | 2 mg/l |
| Mn²⁺ | 2 mg/l |
| RNA | 1.2 g/l |
| CaCl₂·2H₂O | 2 g/l |
| Mi-eki mixture* (Soybean protein hydrolyzate) | 40 ml/l |
| L-glutamic acid | 10 g/l |
| L-methionine | 0.3 g/l |
| pH 7.5 | |
| [Note] *: Product of Ajinomoto Corp. | |

Results obtained are shown in Table 2. As is seen from the table, all the mutants obtained had a lowered ability of liberating phosphoric acid from 5'-GMP but were capable of accumulating riboflavin in greater quantities, compared with the parent strain, Bacillus subtilis AJ12643.

**Table 2**

| Strain | Activity for Liberating Phosphoric Acid From 5'-GMP (µmol/min/mg-protein) | Amount of Riboflavin Accumulated(g/l) |
|---|---|---|
| AJ12643 | 0.127 | 0.12 |
| No. 25 | 0.063 | 0.24 |
| No. 141 | 0.081 | 0.21 |
| No. 189 | 0.075 | 0.28 |
| No. 235 | 0.077 | 0.24 |

One of the mutants obtained above (Bacillus subtilis No. 189) was subjected to mutation-inducing treatment in a similar manner as above, to obtain Bacillus subtilis AJ12644 (FERM BP-3855 ), which had a further lowered activity for liberating phosphoric acid from 5'-GMP. In Table 3 are shown their activity for liberating phosphoric acid from 5'-GMP and the quantity of riboflavin accumulated in culture broths.

**Table 3**

| Strain | Activity for Liberating Phosphoric Acid From 5'-GMP (µmol/min/mg-protein) | Amount of Riboflavin Accumulated(g/l) |
|---|---|---|
| No. 189 | 0.075 | 0.24 |
| AJ12644 | 0.020 | 0.90 |

Riboflavin is synthesized in vivo via a nucleotide, such as 5'-IMP, 5'-GMP and 5'-GTP. Therefore, if a microorganism has a high activity for liberating phosphoric acid from 5'-nucleotides, 5'-nucleotides are dephosphorylated and released out of the cell body. Presumably, the mutants according to the invention, which have a lowered activity for liberating phosphoric acid from 5'-nucleotides, are provided with larger quantities of precursors for synthesizing riboflavin and, as a result, riboflavin could be accumulated in an increased quantity.

In the production of riboflavin using the mutants according to the invention, there can be used an ordinary liquid medium containing carbon sources, nitrogen sources, inorganic salts and, where necessary, minor organic nutrients, as well as nutrients required by the microorganisms. Any carbon sources can be used, provided that they are assimilable by the mutants used. Examples of usable carbon sources include glucose, sucrose, molasses, hydrolyzed products of starch, and the like. Examples of usable nitrogen sources include ammonium sulfate, urea, ammonia and the like. Examples of minor organic nutrients include amino acids, vitamins, fatty acids and nucleic acids. It is also possible to use other materials that contain minor organic nutrients, for example, yeast extract, peptone, casamino acid, and hydrolyzed products of soybean proteins.

Culturing can be carried out with aeration under conditions controlled at a temperature of 30 to 40 °C, preferably at 34 to 37 °C. The pH of the culture medium is maintained at 6.0 to 7.5, preferably 6.5 to 7.0, both at the beginning and during the course of the culturing. For the adjustment of the pH, there can be used an organic or inorganic acid, or an alkaline substance, including urea, calcium carbonate, ammonia gas, and the like. Thus, a marked quantity of riboflavin can be produced and accumulated in the medium in 1 to 5 days.

After the cultivation, riboflavin can be recovered from the medium in accordance with a known procedure. For example, after the removal of the cell bodies, an appropriate quantity of hydrosulfite is added. The resulting mixture is slightly stirred and then subjected to centrifugation at 20 °C to collect crude crystals of reduced-type riboflavin. The crude crystals are suspended into 1N acetic acid solution and dissolved by heating. Impurities are then removed off by filtration, and the filtrate was cooled to allow crystals to precipitate. The crystals are then collected by filtration and dried to give crystals of desired riboflavin.

The invention will further be explained by example.

### Example 1

In a 5 liter fermentation tank was placed 2 liters of medium having a composition set forth in Table 4, and the medium was heated and sterilized at 121 °C for 15 minutes. In this medium was inoculated 100 ml of culture prepared beforehand by cultivating Bacillus subtilis AJ12644 in a liquid medium at 34 °C for 16 hours, and culturing was carried out at a temperature of 34 °C for 3 days with aeration of 0.5 vv m and stirring at 700 rpm, during which the pH of the medium was maintained at 6.5 by use of ammonia. As a result, there was obtained 1.8 liters of culture containing 1.05 g/l of riboflavin. The cell bodies were removed from the culture by centrifugation, and then 20 g of hydrosulfite was added. After being slightly stirred, the mixture was subjected to centrifugation to obtain 1.8 g of crude crystals of riboflavin. The crude crystals were suspended into 500 ml of 1N acetic acid solution and oxidized by adding a small quantity of saturated permanganate. The resulting mixture was then boiled to dissolve precipitated crystals and then hot-filtered. After cooling, precipitated crystals were collected by filtration to give 750 mg of riboflavin. The product was then recrystallized to obtain 560 mg of pure crystals of riboflavin.

**Table 4**

| Components | Concentration |
|---|---|
| Glucose | 80 g/l |
| NH₄Cl | 15 g/l |
| KH₂PO₄ | 0.2 g/l |
| MgSO₄·7H₂O | 0.4 g/l |
| Fe²⁺ | 2 mg/l |
| Mn²⁺ | 2 mg/l |
| RNA | 1.2 g/l |
| CaCl₂·2H₂O | 2 g/l |
| Mi -eki mixture* (Soybean protein hydrolyzate) | 40 ml/l |
| L-glutamic acid | 10 g/l |
| L-methionine | 0.3 g/l |
| pH 7.5 | |
| [Note] *: Product of Ajinomoto Corp. | |

### Advantageous Effect of the Invention

The mutants according to the invention have an improved ability of producing riboflavin and are capable of producing and accumulating a markedly increased amount of riboflavin, compared with the parent strain. The process of the invention is therefore suitable as a process for producing riboflavin in an effective manner at a lower cost.

## Claims

1. A mutant strain of Bacillus subtilis capable of producing riboflavin, said mutant strain having an activity of liberating phosphoric acid from 5'-guanylic acid of not higher than 0.081 µmol/min/mg-protein.

2. A mutant strain of Bacillus subtilis according to claim 1 having the accession no. FERM BP-3855.

3. A mutant strain of Bacillus subtilis according to claim 1 having the accession no. FERM BP-3856.

4. A process for producing a mutant strain of Bacillus subtilis according to any of claims 1 to 3, wherein a riboflavin producing strain of Bacillus subtilis is mutated.

5. A process according to claim 4, wherein said riboflavin producing strain which is to be mutated requires adenine, is GMP reductase deficient and is resistant to purine/8-azaxanthine.

6. A process according to claim 4 or 5, wherein the riboflavin producing strain is mutated by treatment with chemical agents or by irradiation.

7. A process for producing riboflavin by fermentation which comprises:
- culturing in a liquid fermentation medium a mutant of Bacillus subtilis according to any of claims 1 to 3 or a mutant of Bacillus subtilis as obtained by any of claims 4 to 6,
- accumulating riboflavin in the medium, and
- recovering riboflavin from the medium.

## Patentansprüche

1. Zur Produktion von Riboflavin befähigter Mutantenstamm von Bacillus subtilis, der eine Aktivität zur Freisetzung von Phosphorsäure aus 5'-Guanylsäure von nicht mehr als 0,081 µMol/min/mg Protein aufweist.

2. Mutantenstamm von Bacillus subtilis nach Anspruch 1 mit der Hinterlegungsnummer FERM BP-3855.

3. Mutantenstamm von Bacillus subtilis nach Anspruch 1 mit der Hinterlegungsnummer FERM BP-3856.

4. Verfahren zur Herstellung eines Mutantenstammes von Bacillus subtilis nach einem der Ansprüche 1 bis 3, bei dem ein Riboflavin produzierender Stamm von Bacillus subtilis mutiert wird.

5. Verfahren nach Anspruch 4, wobei der zu mutierende Riboflavin produzierende Stamm Adenin benötigt, GMP-Reductase-defizient ist und gegen Purin/8-Azaxanthin resistent ist.

6. Verfahren nach Anspruch 4 oder 5, wobei der Riboflavin produzierende Stamm durch Behandlung mit chemischen Mitteln oder durch Bestrahlung mutiert wird.

7. Verfahren zur Herstellung von Riboflavin durch Fermentation, welches folgende Schritte umfaßt:
- Züchten einer Mutante von Bacillus subtilis nach einem der Ansprüche 1 bis 3 oder einer nach einem der Ansprüche 4 bis 6 erhaltenen Mutante von Bacillus subtilis in einem flüssigen Fermentationsmedium,
- Anreichern von Riboflavin in dem Medium und
- Gewinnen von Riboflavin aus dem Medium.

## Revendications

1. Souche mutante de Bacillus subtilis capable de produire de la riboflavine, ladite souche mutante ayant une activité de libération d'acide phosphorique à partir d'acide 5'-guanylique ne dépassant pas 0,081 µmol/min/mg de protéine.

2. Souche mutante de Bacillus subtilis selon la revendication 1, ayant le numéro de référence FERM BP-3855.

3. Souche mutante de Bacillus subtilis selon la revendication 1, ayant le numéro de référence FERM BP-3856.

4. Procédé de production d'une souche mutante de Bacillus subtilis selon l'une quelconque des revendications 1 à 3, où une souche de Bacillus subtilis productrice de riboflavine est soumise à une mutation.

5. Procédé selon la revendication 4, où ladite souche productrice de riboflavine qui doit être soumise à la mutation nécessite de l'adénine, est déficiente en GMP-réductase et est résistante à la purine/8-azaxanthine.

6. Procédé selon la revendication 4 ou 5, où la souche productrice de riboflavine est soumise à la mutation par traitement par des agents chimiques ou par irradiation.

7. Procédé de production de riboflavine par fermentation, lequel comprend :
- la culture dans un milieu de fermentation liquide d'un mutant de Bacillus subtilis selon l'une quelconque des revendications 1 à 3 ou d'un mutant de Bacillus subtilis obtenu selon l'une quelconque des revendications 4 à 6,
- l'accumulation de riboflavine dans le milieu et
- la récupération de riboflavine dans le milieu.
